# EUROPEAN PATENT SPECIFICATION

(11) **EP 0 782 441 B1**
(45) Date of publication and mention of the grant of the patent: **05.03.2003**
(21) Application number: 95932094.6
(22) Date of filing: 22.09.1995
(51) Int. Cl.: A61K 31/00, A61K 31/40, A61P 29/00

(54) **PHARMACEUTICAL CONTROL OF INFLAMMATION**
ÄNDERUNG DER AKTIVITÄT UND/ODER SPIEGEL VON HÄM-OXYGENASE ZUR BEHERRSCHUNG VON ENTZÜNDUNG
REGULATION PHARMACEUTIQUE DE L'INFLAMMATION

(30) Priority: 22.09.1994 CA 2132690
(43) Date of publication of application: 09.07.1997
(73) Proprietor: William Harvey Research Limited, London EC1 6BQ (GB)
(72) Inventor: WILLIS, Dean, Warwickshire CV9 1HQ (GB); MOORE, Adrian, Richard, Essex IG1 1HG (GB); WILLOUGHBY, Derek, Albert, London N12 0JL (GB)
(74) Representative: Schlich, George William
(86) International application number: GB9502249
(87) International publication number: WO96009038

(56) References cited:
- WO-A-91/04667
- WO-A-92/18112
- WO-A-93/07114
- WO-A-93/13055
- WO-A-94/13252
- GB-A- 2 263 111
- US-A- 5 102 670
- BIOCHEM J., vol. 279, no. pt 3, 1991 pages 891-894, L. CANTONI ET AL. 'Interleukin-1 and tumor necrosis factor induce hepatic haem oxygenase. Feedback regulation by glucocorticoids.'
- NOVOGRODSKY A, SUTHANTHIRAN M, STENZEL KH.: 'Immune stimulatrory properties of metalloporphyrins' JOURNAL OF IMMUNOLOGY vol. 143, no. 12, 15 December 1989, pages 3981 - 3987

## Description

This invention relates to the control of the chronic inflammatory response by the increase of the activity or the amount or both the activity and the amount of heme-oxygenase in the body.

This invention finds in addition to other applications, particular application in the treatment of diseases manifested in or exacerbated by an inflammatory response (such as in the treatment of chronic inflammatory diseases, for example, rheumatoid arthritis), in humans suffering hypersensitivity reactions such as in asthma, in humans suffering conditions in which ROS (Reactive Oxygen Species) have a pathogenic role such as reperfusion injury and atherosclerosis and, in humans having immunosuppressed states resulting from drug treatments or from pathologies such as AIDS.

Chronic inflammatory diseases place a heavy social and economic burden on the resources of many nations, but the number of safe and effective treatments is limited. To date, the major research effort has concentrated on those mediators responsible for the initiation and maintenance of the pathological process. In contrast little attention has been focused on endogenous factors responsible for the resolution of the inflammation.

The main anti-inflammatory pharmaceutical agents are the glucocorticoids and the non-steroidal anti-inflammatory drugs (NSAIDs). Both suffer from well catalogued disadvantages, indeed nearly one quarter of adverse drug reactions reported in the UK are due to NSAIDs. Side-effects of NSAIDs commonly affect the gastrointestinal tract, and also the liver, kidney, spleen, blood and bone marrow. NSAIDs are not effective in treatment of some chronic inflammatory disorders.

Glucocorticoids are powerful anti-inflammatory agents, suppressing both acute and chronic phases of inflammation. They carry the hazard, however, that they also suppress the immune response and can decrease many aspects of essential cell repair processes. Generally, they must be given by injection and are not effective on oral administration.

There thus exists an on-going need for further anti-inflammatory drugs, which can be given orally and which are effective against chronic inflammation.

Conversely, a number of diseases are known in which there is a significant suppression of the inflammatory response. One example is acquired immune deficiency syndrome (AIDS) in which infection by HIV leads typically to such immune-suppression that an infected person dies from a separate, opportunistic, infection, often a bacterial or viral infection rarely fatal in a healthy person.
Stimulation or induction of inflammation in this case could assist in preventing death from non-HIV infections. However, no suitable pharmaceutical is known for this pro-inflammatory purpose.

Heme (ferri-protoporphyrin-IX) plays a vital role in cellular metabolism, functioning as the prosthetic group of hemeproteins (eg. hemoglobin and cytochromes). It's catabolism is a two-step process. The first, and rate limiting reaction, is the production of biliverdin and carbon monoxide by the microsomal enzyme heme-oxygenase. The second step is the production of bilirubin from biliverdin by the cytosolic enzyme, biliverdin reductase.

Heme-oxygenase is found in liver, kidney, spleen and skin, and has also been localised to specific cell types, notably fibroblasts and macrophages. The enzyme exists in at least two isoforms, one constitutive and the other inducible. Heme, heavy metal ions (eg. tin, gold, platinum and mercury) and transition metal ions (eg. iron, cobalt, chromium and nickel) can all induce heme-oxygenase. In addition, heme-oxygenase is induced as part of a generalised stress response to stimuli such as thermal shock (hence the alternative name heat-shock protein 32; hsp32), oxidative stress and cytokines such as interleukin-1 (IL-1), tumour necrosis factor and IL-6. The stress response is seen as beneficial in that it results in protection of vulnerable cell enzymes from inactivation.

Animal biles have been used in traditional Chinese medicine for centuries in the treatment of bronchitis, asthma and other hypersensitivity reactions. More recently, it has been shown that biliverdin and bilirubin scavenge reactive oxygen species (ROS) which can have a range of proinflammatory effects including inactivation of protease inhibitors, depolymerisation of hyaluronic acid to angiogenic fragments and alteration of proteins to give rise to endogenous antigens.

Abraham et al., Int. J. Biochem. 20(6): 543-558 (1988) provides a general review of heme-oxygenase and its role in mammalian physiology including regulation of its activity by endogenous and exogenous factors. The interaction of this enzyme with NADPH-cytochome P450 reductase is discussed, as is the role of heavy metal ions in inducing heme-oxygenase activity. Metal Porphyrins are taught as inhibitors. At p. 548 of the reference, the author states:
"In general, heme-oxygenase activity increases when cells are under stress or in a disease state. In fact, the enzyme may be regarded as a red light signalling the occurrence of abnormality."

Sacerdoti et al., Science 243: 388-390 (1-20-89) asserts that hypertension was linked to kidney cytochrome P450 levels, and that enzyme could be depleted by inducing heme-oxygenase production. This was done by treatment with Co²⁺.

McCarty (Chemical Abstract, 100: 1864024 (1984) purports to teach the effect of selenium in reducing leukotrienes which are alleged to be prominent mediators of hyper-sensitivity and inflammation. McCarty, in fact, teaches the administration of a dietary adjuvant per os.

U.S. Patent No. 5102670 relates to the reduction of ocular swelling by administering a heme-oxygenase inducer to the swollen eye of an individual to increase production of heme-oxygenase to reduce the amount of 12(R) hydroxyeicostetraenoic acid (12(R)-HETE) present in the eye as well as 12(R) Dihydroxyeicosatrienoic acid (12(R)-DIHETE) also present in the eye. The scheme presented in the patent for reducing corneoconjunctival swelling is thus dependent on the reduction of the arachidonic acid metabolites 12(R)HETE and 12(R)DIHETE found mainly in the eye. In particular, it is explained that the therapeutic effect is primarily on maintaining the blood - aqueous barrier, a barrier not seen other than in the eye. Thus, the teachings in the patent relate solely to the eye and have very little other applicability.

During about the same time period Messrs. George S. Drummond and Hallah Kappas of The Rockefeller University, Drummond, N.Y. were involved together and with others in research relating to heme-oxygenase. The result of the research appears embodied, at least in part, in U.S. Patents No.'s 4657902; 4699903; 4684637; 5010073 and 5223494.

U.S. Patent No. 4657902 purports to teach the use of the novel compound tin mesoporphyrin and compositions containing it to inhibit heme metabolism in mammals, to control the rate of tryptophan metabolism in mammals, and to increase the rate at which heme is excreted by mammals.

The patent disclosure provides as follows at Column 1, lines 22-36:
"In mammals and other vertebrates heme is oxidatively degraded by heme-oxygenase to form the open chain tetrapyrrole biliverdin. In mammals biliverdin is reduced to bilirubin by biliverdin reductase. In liver bilirubin is converted to the mono- and di-glucuronide conjugates by the hepatic glucuronyl transferase system prior to its excretion.
Bilirubin is a toxic compound, but normally this toxicity is not manifest since bilirubin is rapidly bound to plasma proteins, transported to liver, conjugated and excreted. However, in the newborn, high undesirable concentrations of bilirubin exist in serum and may produce neurotoxicity. The intractable neurological syndrome known as "kernicterus" is the most severe manifestation of bilirubin toxicity."

The inventors, therefore, provide at column 3, lines 43-48:
"It has now been discovered that the compound Sn(tin)-mesoporphyrin (SnMP) can be employed in the treatment of mammals including humans in need of such treatment to decrease the rate of heme metabolism, to increase the rate at which heme is excreted and to control the rate of tryptophan metabolism in the liver."

U.S. Patent No. 4699903 purports to teach a method of increasing the rate at which heme is excreted by a mammal in need of increased disposal of heme by administering tin diiododeuteroporphyrin.

U.S. Patent No. 4684637 discloses methods for decreasing the rate of metabolism for decreasing the rate of metabolism of heme in mammals by administration of tin or chromium protoporphrins IX.

U.S. Patent No. 5010073 purports to relate to liposomal metalloporphyrin preparations for targeting the spleen for inhibition of heme-oxygenase activity in the spleen.

U.S. Patent No. 5223494 purports to teach a method for inhibiting heme-oxygenase activity in the intestine by administering mesoporphyrin for reducing the absorption of iron from foodstuffs by animals in need of such prevention.

This latter group relates to inhibiting heme-oxygenase in selected body tissues by targeting those body tissues (for example, the spleen or intestine) for specified purposes.

Cantoni et al. (Biochem. J. (1991) 279, pp891-894) describes an investigation into the role of IL-1 and TNF in the pathway of LPS-mediated induction of heme-oxygenase. Cantoni et al. show that administration of these strongly pro-inflammatory cytokines can cause elevation of heme-oxygenase activity in the livers of mice.

WO-A-9413252 relates to treatment of epidermal conditions characterised by excessive cellular exfoliation and/or hyperkeratinization with topical preparations of vitamin B12 in a sufficient amount to induce heme-oxygenase in the affected epidermal tissue.

Heat shock proteins (HSPs) or stress proteins are a group of proteins that are among the most highly conserved and abundant proteins in the biosphere. Although many of the isoforms of the proteins are expressed under normal physiological conditions and serve vital roles in the cell, they are greatly upregulated by factors which threaten the integrity of the cell. These factors include heat and cold shock, oxygen radicals, heavy metals, hypoxia, infection, ethanol, ionophores and thiol reactive agents. This increase in the cell's concentration of HSPs leads to the cell being transiently resistant to an otherwise lethal insult, and unresponsive to some biological mediators.

The role of HSPs in pathological conditions has attracted much attention. Immune responses to HSPs can be highly cross-reactive and even auto-reactive due to their extensive inter-species amino acid homology. Immune responses to HSPs have already been implicated in adjuvant arthritis in the rat, pristane arthritis in the mouse, diabetes mellitus in the non-obese diabetic mouse, rheumatoid arthritis, systemic lupus erythematosus, atherosclerosis and in tumour surveillance. Therefore, it appears that HSPs may have a paradoxical effect in pathological conditions, having a cyto-protective effect on cells and tissue in stressful environments, but eliciting a detrimental immune response in some autoimmune diseases.

Inflammation involves the sequential release of various mediators including vasoactive mediators, chemoattractants, cytokines, prostaglandins, free radicals and proteases. In rheumatoid arthritis, a chronic inflammatory disease, HSPs are upregulated in the synovial lining of patients. However, their role in inflammation has yet to be completely elucidated.

Objects of this invention are to provide improved (or at least alternative) means for control of inflammation, in particular treatment of chronic inflammation.

We have now discovered that by controlling the amount or the activity or both the amount and the activity of heme-oxygenase in the body, either (1) a generalized anti-inflammatory effect or response is obtained wherein heme-oxygenase is induced, or (2) a general inflammatory response is obtained wherein heme-oxygenase is suppressed (or inhibited).

According to one use of our invention, chronic inflammation is treated by administration of a compound that induces heme-oxygenase or stimulates or increases the activity of heme-oxygenase. The treatment is systemic. This treatment induces heme-oxygenate production and/or stimulates its activity; specific medical applications include the treatment of chronic inflammatory diseases for example, rheumatoid arthritis, the treatment of hypersensitivity reactions such as in asthma, and, the treatment of injury, atherosclerosis and infarction.

For the treatment above a pharmaceutical composition that comprises a compound that induces, stimulates or increases the activity of heme-oxygenase, in combination with a pharmaceutically acceptable carrier can be used. The composition is in the form of a solution for injection, an orally acceptable composition.

A suitable solution for injection includes a sterile, saline-containing solution, preferably containing saline at approximately physiological concentration.

For oral administration, the composition can be in a solid form-such as a tablet, a pill or a powder for suspension in water or for dissolution in water. The preparation of such a solid composition will be known to a person of skill in this art. It is an option to supplement the oral composition with a taste enhancing agent, to make the composition more palatable, or at least less unpleasant by mouth. Suitable taste enhancers includes sweeteners, flavourings and agents that mask or reduce any unpleasant or undesirable taste in the active component of the pharmaceutical.

The use of the invention thus provides an anti-inflammatory pharmaceutical. It can be given orally, according to the particular compound chosen, and initial tests have demonstrated useful anti-inflammatory activity. The products of heme-oxygenase are believed to have multiple effects in the progression of inflammation, whereas the products of the enzyme targeted by most NSAIDs, cycloxygenase, have fewer effects, so the compositions present a pharmaceutical activity potentially more powerful than that seen in NSAIDs.

Another embodiment is an anti-inflammatory composition comprising a heme-oxygenase inducer or stimulator and a pharmaceutically acceptable carrier.

A further embodiment is the use of a compound that induces, stimulates or increases the activity of heme-oxygenase in the manufacture of a medicament for the treatment of chronic inflammation.

Agents suitable for inducing heme-oxygenase include prostaglandins of the A series; analogues, derivatives, complexes and conjugates of prostaglandins of the A series that are agonists of prostaglandin A receptors; agonists of prostaglandin A receptors; vitamin B₁₂; hemin; and fragments, sub-units, conjugates, analogues, derivatives and complexes thereof that retain heme-oxygenase inhibiting activity. Suitable dosage amounts are from 0.1 to 30 µmoles/kg of body weight of for example, a human. For example, where hemin (FePP) is used, suitable dosage amounts would be from about 0.06mg hemin to about 24 mg hemin per kilogram of body weight.

In specific embodiments of the use of the invention, inflammation is treated by inducing or stimulating a heme-oxygenase in mononuclear cells, such as in particular an inducible heme-oxygenase found in monocytes and macrophages. This is advantageous when treating chronic inflammation, typically medicated by and characterised by a large population of these cells at an inflammation site.

Preferably, the inducer is administered in a suitable pharmaceutically acceptable vehicle in any suitable manner (for example, sterile water, saline (sterile)). A buffered, isotonic aqueous solution having a pH of between about pH7 and pH8 is suitable. Lyophilised preparations are also be suitable. The lyophilised preparations may be reconstituted with sterile water.

Diseases comprising chronic inflammation that can be treated according to the first aspects of the invention include chronic inflammatory joint disease - eg rheumatoid arthritis, chronic inflammatory bowel disease - eg Crohn's disease, respiratory inflammatory disease - eg asthma, chronic inflammation of the brain - eg multiple sclerosis, and inflammation of soft tissues - eg tendonitis.

Dosage amount(s) can be given over a period of time. However, the effects of compounds used to date appear to peak 24 hours after administration of each dosage amount and regress at 48 hours after administration.

The formulation can be in a unit dosage form (for one dosage) or may be packaged as a multiple formulation composition from which suitable dosage amounts can be extracted and used. For example, a solution in a vial may provide the multiple formulation composition from which the dosage amounts can be taken and administered. Compositions and uses of the first aspect of the invention are optionally supplemented by one or both of (1) a NSAID, and (2) a NOS (nitric oxide synthetase) inhibitor.

The invention will now be illustrated in specific embodiments with reference to the figures in which:-
Figure 1 illustrates the development of an acute inflammation at 2, 6 and 24 hours after injection of carrageenin into the rat pleural cavity (open boxes - Cell exudate, closed boxes - Total cells, n = 6 each time point);
Figure 2 illustrates the measurement of HO activity as nmol bilirubin /60'/mg protein against time in hours during acute inflammation (n=6 each time point);
Figure 3 is a photograph showing HSP detection by Western blot analysis, with a peak at 24 hours;
Figure 4 shows the development of acute inflammation in hours after injection of carrageenin into the rat pleural cavity (□ Exudate volume, ■ Total inflammatory cells, n = 10);
Figure 5 shows heme-oxygenase (HO) activity of inflammatory cells against time (hours) during carrageenin pleurisy, the activity is expressed as pmoles bilirubin/mg protein/hour, n=9 (results are expressed as the mean ± s.e.mean, ■ Peripheral blood mononuclear cells (PBMNs), □ Time course of inflammatory cells);

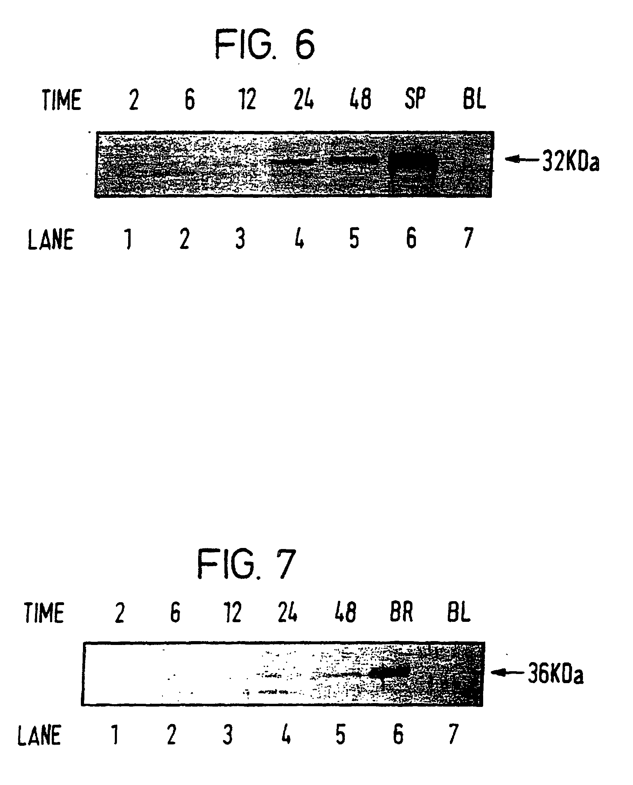

Figures 6 and 7 show Western blot analysis for heme-oxygenase isoforms: Fig. 6) Analysis of inflammatory cell pellet for HO-1. Lanes, inflammatory cells taken at 2, 6, 12, 24 and 48 hours, spleen homogenate (SP), PBL (BL), Fig. 7) Analysis of inflammatory cell pellet for HO-2. Lanes, inflammatory cells taken at 2, 6, 12, 24 and 48 hours, brain homogenate (BR), PBL (BL);
Figures 8-10 show immunolocalization of inflammatory cell smears for heme-oxygenase 1 confirming increased positive staining for heme-oxygenase-1 protein in mononuclear cells: Fig. 8) Normal rabbit serum (control), Fig. 9) 6 hour inflammatory cell smear, Fig. 10) 48 hour inflammatory cell smear;

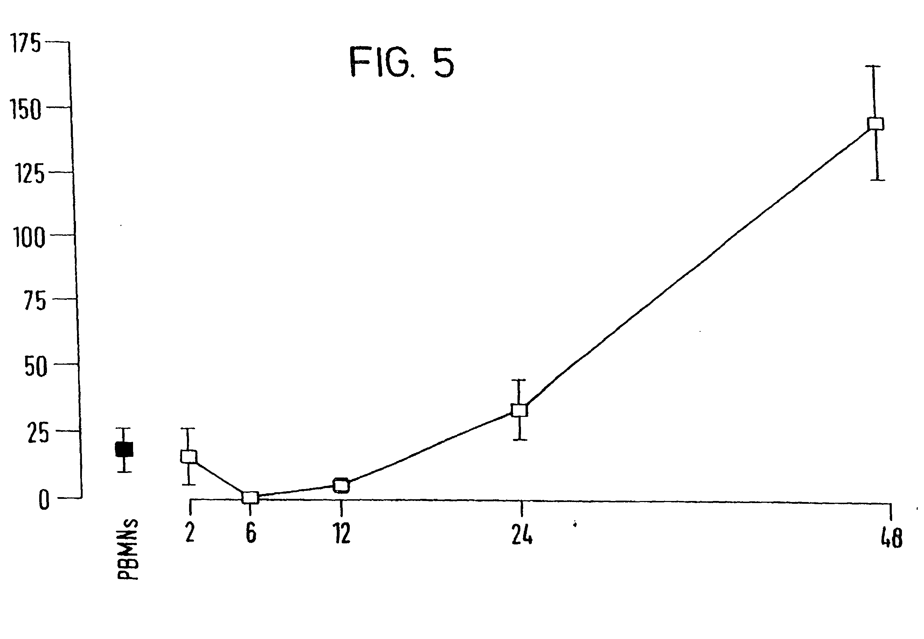

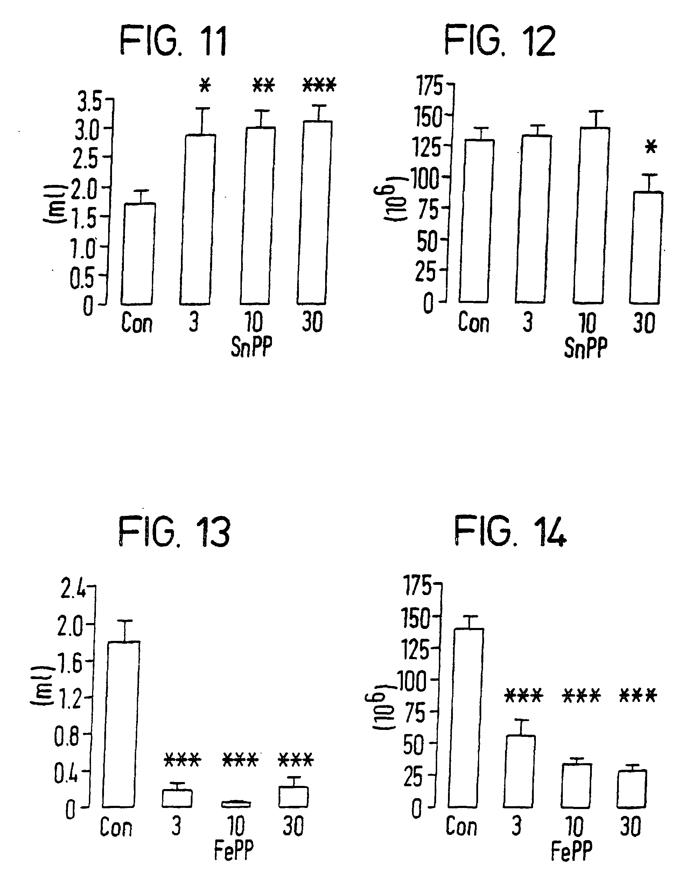

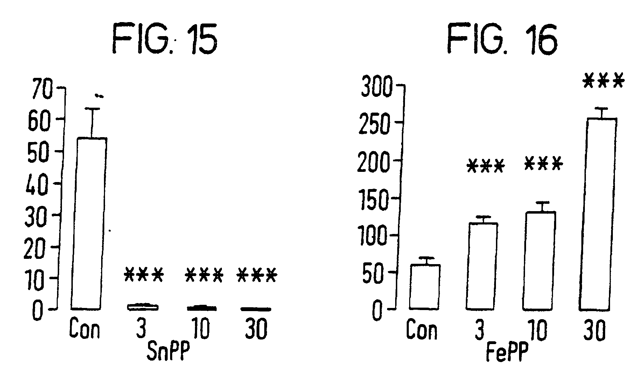

Figures 11-16 show the effect of heme-oxygenase (HO) modulators on inflammation and HO activity: Effect of tin protoporphyrin dichloride (SnPP) on acute inflammation at 24 hours (Fig. 11 - exudate volume, Fig. 12 - cell number), Effect of ferriprotoporhyrin IX dichloride (FePP) on acute inflammation at 24 hours (Fig. 13 - exudate volume, Fig. 14. - cell number), Effect of SnPP (Fig. 15) and FePP (Fig. 16) administration on HO activity in 24 hour inflammatory cells, inflammatory cells were collected after treatment with the above porphyrins and HO activity measured as for Figure 5, n=6. ^{*} *P*<0.05, ^{****} *P*<0.01 and ^{***} *P*<0.001;

### Example 1

In the following example, we examined the expression and possible involvement of heme-oxygenase in acute and chronic inflammation The highly inducible isoform of heme-oxygenase-1 has recently been classified as an HSP (HSP32). This is the rate limiting enzyme in the catabolism of the prosthetic group of heme proteins. The major product of this reaction, bilirubin, is a powerful free radical scavenger and may represent an endogenous mechanism by which cells protect themselves from free radical damage. Carbon monoxide (CO) is also produced by this reaction. Due to the structural similarities to nitric oxide a major mediator of inflammation, and the ability of both molecules to bind heme proteins the role of CO in inflammation raised an intriguing question for which we sought an answer.

In this embodiment, the rat carrageenin pleurisy model of acute inflammation has been used to determine the expression and activity of HSP32 in inflammatory cells. Markers of inflammation, cell exudate and cell number progressively increased over 2, 6 and 12 hours peaking at 24 and regressing at 48. The inflammation was dominated by polymorphonuclear leucocytes (PMNs) at the early time points, with an increase in the monocyte population as inflammation progressed. The increase in monocytes was associated with an increase in HSP32 protein levels determined by Western blot analysis, intensely labelled bands at 24 and 48 hours. Immunohistochemistry confirmed that HSP32 protein was present in monocytes. Heme-oxygenase (HO) activity was also assayed at 2, 6 and 24 hours, the later time point being the only one to show activity. The effects of modulating HO activity with hemin (an inducer of HSP32 expression) and tin Protoporphyrin-IX (synthetic analogue of heme which inhibits HO activity) was then examined. The inducer or the inhibitor had no significant effect on inflammation at 6 hours compared to controls. However, at 24 hours the inhibitor significantly increased cell exudate by 135% (p<.001 Mann-Whitney), whereas the inducer of HSP32 decreased cell exudate and total cells by 83% (p<.001) and 50% (p <.001) respectively. The effects of these compounds thus corresponds to the temporal expression of HSP32 in this model of acute inflammation.

### Materials and Methods

### Animals & Drugs

Male Wistar rates 200±20 grams (Tuck & Sons Ltd., Essex, UK) were used for inflammation. Animals were administered either with 15mg/kg Ferriprotoporphrin IX chloride (FePP) by intravenous injection - 18 hours before inflammation or with 2 doses of 40µmoles/kg Tin protoporphyrin dichloride (SnPP) by sub-cutaneous injection - 18 hours and at the time of inflammation induction (porphyrins were obtained from Porphyrin Products Inc., Logan, Utah). Drugs were prepared in 0.1 N NaOH and mixed 1:1 with saline, these were then adjusted to pH 7.4, drug vehicle. The total volume injected was 0.2ml. Control animals received vehicle only.

### Induction of Pleurisy

Carrageenin pleurisy was induced in rats and cell pellet prepared as previously described by Tomlinson et al.

### HO Activity

HO Activity in the cell pellets was measured in post mitochondrial supernatant by quantifying the generation of bilirubin (Jollie DR et al, Arch Biochem Biophys, 1985, 240, pages 51-59), biliverdin reductase was substituted with rat hepatic cytosol (3mg/ml). Protein was estimated by the Bradford method using BSA as standard.

### Western Blot Analysis

Cell pellets were lysed by the addition of protease inhibitory buffer containing 1% Triton X100 and boiled (10 min.) with gel loading buffer (Tris, 50mM; SDS, 10%; glycerol, 10%; 2-mercapthoethanol, 10% bromophenol blue, 2mg/ml) in a ratio of 1:1 and centrifuged at 10,000g, for 10 min. The protein concentrations of supernatants were determined as above, and total protein equivalents (20µg) for each sample separated on 10% sodium dodecyl sulfide-polyacrylamide mini-gels (Hoefer; Staffordshire, UK) using the Laemmli buffer system and transferred to polyvinylidene difluoride membranes (Millipore, Hertfordshire, UK). Non-specific IgGs were blocked with 5% dried milk protein and incubated with a polyclonal antibody to HSP32 1:1000 dilution (Stressgen Crop., Victoria, Canada). Bands were detected with an amplified alkaline phosphatase kit (Sigma Co. Poole, UK) and developed with nitroblue tetrazolium (NBT)-5-bromo-4-chloro-3-indonyl phosphate (BCIP). Rainbow marker and pre-stained blue protein markers were used for molecular weight determinations.

### Statistics

Results are expressed as the mean ± s.e. mean. Statistical analysis was determined by Mann-Whitney U-test. with a *P* value of <0.05 considered significant.

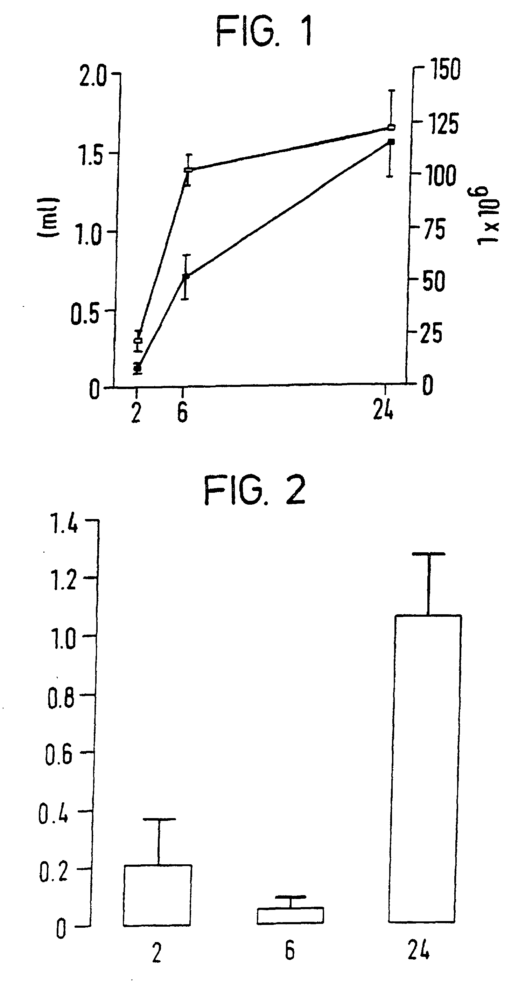

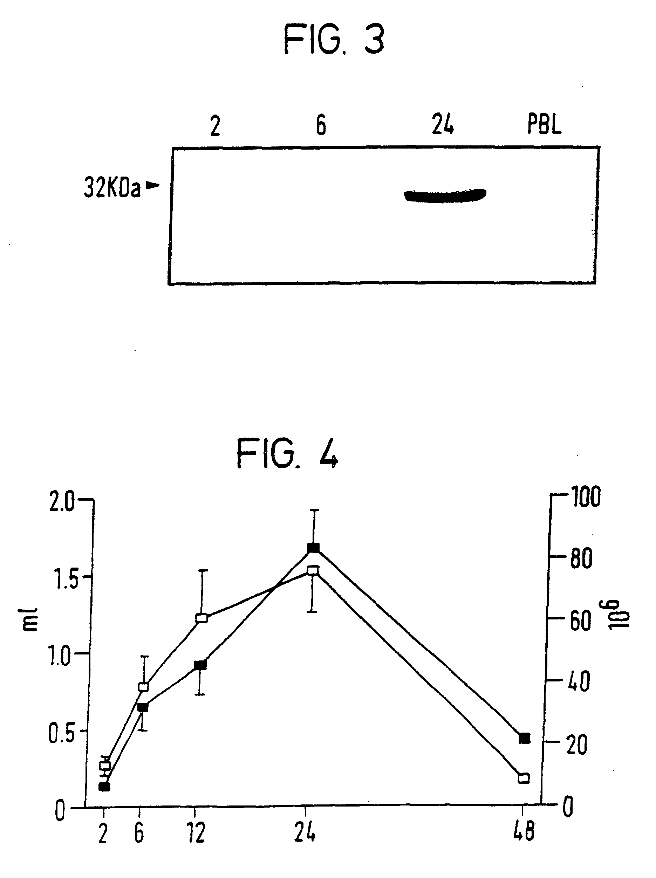

Figure 1 shows the development of exudate volume and cell number during inflammation in the pleural cavity; these were both maximal 24 hours after injection of the carrageenin. A significant increase in HO activity in the exudate cell pellet, Figure 2, and the detection of HSP32 protein by western blot analysis, Figure 2, and Figure 3 (photo), coincided with this peak in inflammation. The effects of the HO inhibitor SnPP and inducer FePP on carrageenin inflammation were then investigated (Table 1). Exudate volumes and cell numbers at 6 hours were not significantly modified by pre-treatment with HO inhibitor or inducer. However, 24 hours after initiation of the inflammation SnPP increased exudate volume by 128% as compared to vehicle control (P<0.001) whereas FePP decreased exudate volume by 73% and cell number by 50% compared to vehicle control (both P<0.O01).

The effect of heme-oxygenase inhibitor Tin protoporphyrin dichloride (SnPP) and inducer ferriprotoporphyrin IX chloride (FePP) on acute inflammation was examined. Treatment animals received either 2 doses of 40µmoles/kg SnPP s.c. -18 and 0 hours before induction of inflammation, or 1 doses of 15mg/kg FePP i.v - 18 before inflammation. Treatment animals were compared to control animals which had received vehicle only by the appropriate route. ***P< 0.001

These results demonstrate for the first time the induction of HSP32 expression and HO activity in inflammatory cells from a model of acute inflammation. The increase in HO activity reported above is associated with an increase in macrophages population in the pleural cavity. Immunohistochemistry demonstrated strong positive immunoreactivity of inflammatory macrophages but not peripheral monocytes for HSP32 in this model (data not given). The effect of pre-stimulating or inhibiting HO activity significantly decreases or increases inflammation respectively at 24 hours. This indicates that the major effect of HO modulators is on inflammatory macrophages at 24 hours, with no significant effect at 6 hours when the inflammatory site is dominated by polymorphonuclear leucocytes. An increase in HO activity achieved by the invention represents a novel therapy for the treatment of chronic inflammatory disease and, conversely, a decrease in activity will be of benefit in those individuals with an impaired inflammatory response.

### Example 2

### Methods

### Drugs Administration

Animals received 2 doses of either 3, 10 or 30µmoles/kg SnPP (a HO inhibitor) or vehicle control -18 hours and at the time of inflammation induction. Animals received 1 dose of either 3, 10 or 30µmoles/kg FePP (a HO inducer) or vehicle control -18 hours before induction of inflammation, n=9.

### Induction of pleurisy.

Male Wistar rats 200+20 grams were anaesthetized with halothane and 150ul of a 1% carrageenin solution in 0.9% NaCl injected into the pleural cavity. Exudates were collected 2, 6, 12, 24 and 48 hours after injection of the irritant by pleural lavage with 1ml protease inhibitory buffer containing: trisodium citrate 3.15%, phenylmethylsulfonyl fluoride 1mM (Sigma Chemical Co., Poole, UK), pepstatin A 1.5mM (Sigma) and leupeptin 0.2mM (Sigma) in 10mM phosphate buffered (PBS) pH 7.3 . Any exudates with blood contamination were rejected. Cells were counted using a Coulter counter, model DN (Coulter Electronics Ltd., Luton, UK). Each sample was centrifuged at 800g for 10 minutes and the resultant cell pellet prepared as outlined below.

### Immunohistochemistry.

Peripheral blood and exudate cell samples were smeared onto poly-L-lysine coated slides and allowed to air dry. Prior to immunolabelling or histological staining, smears were fixed in 4% paraformaldehyde in 0.1M phosphate buffer for 1 hour. Endogenous peroxidase were quenched with 0.3% H₂O₂ in methanol and sections washed with 0.1 % Triton X100 in PBS. Non-specific binding of IgGs was blocked using NGS 1:50 in 0.1 % essentially globulin free BSA in PBS. The sections were incubated overnight with polyclonal anti-HO-1 (1:200) or polyclonal anti-HO-2 (1:1000) at 4°C, washed and incubated for a further 30 minutes with biotinylated goat anti-rabbit secondary antibody. Following a further 30 minutes incubation with Vectastain ABC horseradish-peroxidase (Vector Labs, Peterborough, UK), the substrate 0.05% diaminobenzidine tetrahydrochloride (Sigma) in 0.05M Tris buffer pH 7.6 was added for the appropriate time period (5-10 mins). This resulted in positive immunoreactivity labelling brown. Primary antiserum was replaced with NGS, as a negative control. Morphological observations were confirmed using the routine histological stain, haematoxylin and eosin.

### Statistics

Results are expressed as the mean ± s.e.mean for (*n*) experiments. Statistical analysis was determined Student's unpaired *t* test, with a *P* value of <0.05 considered significant.

### Heme-oxygenase Assay

HO activity is assessed as previously described by Sierra ES et al, *Anal. Biochem.* 1992; 200:27-30. Cells are lysed by sonication for 10 seconds in protease inhibitory buffer. Heme-oxygenase is assayed as previously described. The reaction mixture (15µl) consists of 11.2µM [¹⁴C] heme specific activity 54 Ci/mol), 1mM NADPH, 2mM glucose-6-phosphate,0.1 units of glucose-6-phosphate dehydrogenase, 3mg/ml liver cytosolic protein and 50-100µg of sample protein. The reaction is started by the addition of the heme, incubated at 37°C for 30 minutes and stopped by the addition of excess cold heme and bilirubin followed by placing on ice. 2µl of reaction mixture is spotted twice onto a silica gel thin-layer chromatography sheet. The chromatogram is developed using a 20:1 dilution of chloroform:acetic acid. Spots corresponding to hem and bilirubin are were cut out and placed in 10ml of scintillation fluid to be counted. Protein determination were carried by the Bradford method (Bradford MM et al *Anal. Biochem.* 1976; 72:248-254), BSA was used as protein standard. The activity is expressed as pmoles bilirubin/mg protein/hour, n = 9. Results are expressed as the mean ± s.e.mean.

### Results

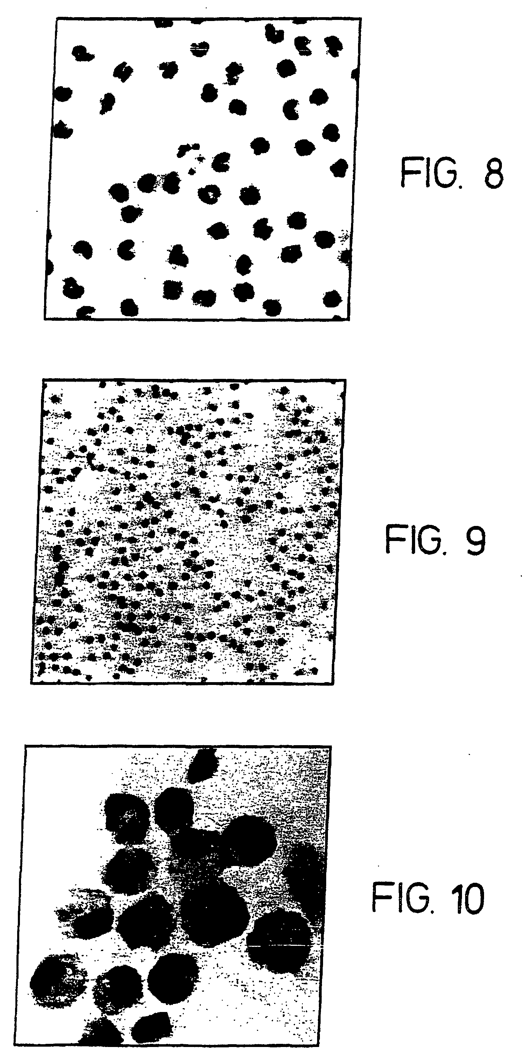

Injection of carrageenin into the pleural cavity of a rat resulted in the development of an acute pleurisy which was maximal at 24 hours as assessed by inflammatory cell number and exudate volume (Fig 4). At 48 hours the inflammatory lesion had virtually resolved, with cell number and exudate volume reduced to 25% and 11% respectively of levels seen when inflammation was maximal. The early part of the inflammation was characterised by influx of polymorphonuclear cells (PMNs) into the pleural cavity, with an increasing percentage of mononuclear cells (MNs) occurring at 24 and 48 hours [Table 2]. HO activity dramatically increased as the inflammation progressed, the highest activity being recorded when the inflammation was resolving (Fig 4) when MNs dominated the inflammatory lesion. Western blot analysis confirmed that there was a dramatic increase in HO-1 protein levels (data not shown). In inflammatory cell smears HO-1 immunoreactivity was specifically localized to mononuclear cells with PMNs showing little or no staining. The number of positively stained cells increased as the inflammation progressed (Figs. 8-10). A similar staining profile with immunoreactivity being localised to mononuclear cells was observed with polyclonal antibodies to HO-2.

These results indicated that there is an increase in HO expression and activity in inflammatory cells. To investigate whether increased HO activity was not only associated with tissue protection but also involved in resolving acute inflammation we used an HO inhibitor, tin protoporphyrin (SnPP), administered sub-cutaneously 18 hours before and at the time of carrageenin injection, and an HO inducer, ferriprotoporphyrin IX chloride (FePP), given sub-cutaneously 18 hours before induction of the inflammation (Maines MD et al., *FASEB J.* 1988; 2:2557-2568).

Neither SnPP or FePP treatments had an effect on the inflammatory lesion as compared to vehicle control 6 hours after injection of the carrageenin, the PMNs phase of inflammation, (data not shown). However, 24 hours after injection of carrageenin, when there was a dominance of MNs, animals receiving SnPP (the HO inhibitor) showed a dose dependant *increase* in inflammatory exudate, with animals dosed 3µmoles/kg SnPP increasing exudate volume by 70% compared to vehicle control (Fig 11). It was noted that the highest dose of SnPP (30µmoles/kg) increased exudate volume further but resulted in a decrease in inflammatory cell number (Fig. 12), the likely explanation the later being a toxic effect. In contrast, animals receiving FePP, the HO inducer, showed a dose dependant *suppression* of inflammatory cell number and exudate volume (Fig 13 and Fig. 14), all the dosages used significantly reduced inflammation with the lowest dose of FePP (3µmole/kg) reducing inflammatory cell number and exudate volume by 90% and 55% respectively compared to vehicle control.

The degree of suppression achieved is greater than that seen with optimal doses of conventional anti-inflammatories or steroids in this model. Analysis of the inflammatory pellets for HO activity confirmed reduction of HO activity with SnPP and increase after treatment with FePP (Fig 15 and 16). These finding would support the idea of HO activity being important in the resolution of acute inflammation. The data would also suggest that the main mode of action of SnPP and FePP is via modulation of HO activity and not other mechanisms, as the phenomena appears dose dependant, and the opposing effects of the porphyrins is only observed when HO is expressed.

In conclusion, the findings of this example implicate a direct involvement of HO in the resolution of acute inflammation. The presence of HO in the MNs, a cell type closely associated with chronic inflammation, would support the concept of this enzyme as a modulator of the chronic inflammatory response, and therefore a target for therapies according to the various aspects of the present invention.

**Table 1**

| **Treatment Groups** | **Exudate Volume (ml)** | **Total Cells 10**^{**6**} |
|---|---|---|
| ***6 hours*** | | |
| Vehicle injected s.c (n = 27) | 1.26 ± 0.10 | 72 ± 7 |
| SnPP injected s.c (n = 26) | 1.59 ± 0.12 | 67 ± 6 |
| Vehicle injected i.v. (n = 23) | 1.29 ± 0.15 | 69 ± 8 |
| FePP injected iv.v (n = 26) | 0.99 ± 0.11 | 50 ± 8 |

| ***24 hours*** | | |
|---|---|---|
| Vehicle injected s.c (n = 29) | 0.79 ± 0.12 | 63 ± 6 |
| SnPP injected s.c (n = 24) | 1.80 ± 0.21 | 58 ± 6 |
| Vehicle injected i.v. (n = 22) | 1.37 ± 0.16 | 90 ± 6 |
| FePP injected iv.v (n = 25) | 0.37 ± 0.07 | 45 ± 6 |

**Table 2**

| **Time** | **Cell type** | **% of total cell number**^{*****} | **% + ve for** **HO-1**^{**#**} | **% + ve for** **HO-2**^{**#**} |
|---|---|---|---|---|
| 2 hours | PMNs | 86 | 0 | 0 |
| | MNs | 14 | 25 | 45 |
| 6 hour | PMNs | >99 | 0 | 0 |
| | MNs | < 1 | N.D. | N.D. |
| 12 hours | PMNs | >99 | 0 | 0 |
| | MNs | < 1 | N.D. | N.D. |
| 24 hours | PMNs | 62 | 0 | 0 |
| | MNs | 38 | 36 | 41 |
| 48 hours | PMNs | 30 | 0 | 0 |
| | MNs | 70 | 89 | 46 |

| | | | | |
|---|---|---|---|---|
| **Immunocytochemical analysis of inflammatory cell smears.** ^{*} Relative percentage of polymorphonuclear cells (PMNs) and mononuclear cell (MNs) in inflammatory cell smear. | | | | |
| ^{#} Percentage of PMNs and MNs present, which are positive for heme-oxygenase isoforms. N.D. insufficient cells present for analysis. | | | | |

Thus, control of chronic inflammation according to the invention is achieved, in embodiments of the invention, by control of HO.

As many changes can be made to the embodiment of the invention without departing from the scope of the invention, it is intended that all material contained herein be interpreted as illustrative of the invention and not in a limiting sense.

## Claims

1. Use of a compound that increases activity, or amount, of heme-oxygenase (HO) in a mammal, in the manufacture of a medicament for treatment of chronic inflammation.

2. Use according to Claim 1 of a compound that increases HO activity in the manufacture of a compound for the systemic treatment of chronic inflammation.

3. Use according to Claims 1 or 2 of a compound that induces HO.

4. Use according to Claim 3 of a compound that induces HO-1.

5. Use according to any previous Claim of a compound that increases HO activity in the manufacture of a medicament for treatment of a disease selected from rheumatoid arthritis, a chronic inflammatory bowel disease, multiple sclerosis, asthma, airways inflammatory disease, tendonitis and chronic inflammation in the brain.

6. Use according to any previous Claim, wherein the compound is FePP (hemin).

## Patentansprüche

1. Verwendung einer Verbindung, die die Aktivität oder Menge der Hämoxygenase (HO) in einem Säuger erhöht, bei der Herstellung eines Medikament zur Behandlung chronischer Entzündungen.

2. Verwendung gemäß Anspruch 1 einer Verbindung, die die HO-Aktivität erhöht, bei der Herstellung einer Verbindung für die systemische Behandlung von chronischen Entzündungen.

3. Verwendung gemäß Anspruch 1 oder 2 einer Verbindung, die HO induziert.

4. Verwendung gemäß Anspruch 3 einer Verbindung, die HO-1 induziert.

5. Verwendung nach einem der vorstehenden Ansprüche einer Verbindung, die HO-Aktivität erhöht, bei der Herstellung eines Medikaments zur Behandlung einer Erkrankung, ausgewählt unter rheumatoider Arthritis, einer chronisch entzündlichen Darmerkrankung, Multipler Sklerose, Asthma, entzündlichen Atemwegserkrankungen, Tendonitis und chronischer Entzündungen im Hirn.

6. Verwendung nach einem der vorstehenden Ansprüche, worin die Verbindung FePP (Hämin) ist.

## Revendications

1. Utilisation d'un composé qui augmente l'activité ou la quantité d'hème oxygénase (HO) chez un mammifère, dans la fabrication d'un médicament destiné au traitement de l'inflammation chronique.

2. Utilisation selon la revendication 1 d'un composé qui augmente l'activité HO dans la fabrication d'un composé pour le traitement systématique de l'inflammation chronique.

3. Utilisation selon les revendications 1 ou 2 d'un composé qui induit l'HO.

4. Utilisation selon la revendication 3 d'un composé qui induit l'HO-1.

5. Utilisation selon l'une quelconque des revendications précédentes d'un composé qui augmente l'activité HO dans la fabrication d'un médicament destiné au traitement d'une maladie choisie parmi l'arthrite rhumatoïde, l'affection abdominale inflammatoire chronique, la sclérose en plaques, l'asthme, les maladies inflammatoires des voies aériennes, les tendinites et les inflammations chroniques du cerveau.

6. Utilisation selon l'une quelconque des revendications précédentes, dans laquelle le composé est FePP (hémine).
